# FASCICULE DE BREVET EUROPEEN

(11) **EP 4 514 214 B1**
(45) Date de publication et mention de la délivrance du brevet: **26.11.2025**
(21) Numéro de dépôt: 23720052.2
(22) Date de dépôt: 12.04.2023
(51) Int. Cl.: A61B 5/021, A61B 5/024, A61B 5/00

(54) **MÉTHODE D'ÉVALUATION D'UNE PRESSION DE CONTACT ENTRE UN CAPTEUR OPTIQUE ET LA PEAU D'UN UTILISATEUR ET DISPOSITIF ASSOCIÉ**
VERFAHREN ZUR BEWERTUNG EINES KONTAKTDRUCKS ZWISCHEN EINEM OPTISCHEN SENSOR UND DER HAUT EINES BENUTZERS UND ZUGEHÖRIGE VORRICHTUNG
METHOD FOR EVALUATING A CONTACT PRESSURE BETWEEN AN OPTICAL SENSOR AND THE SKIN OF A USER AND ASSOCIATED DEVICE

(30) Priorité: 25.04.2022 FR 2203817
(43) Date de publication de la demande: 05.03.2025
(73) Titulaire: Withings, 92130 Issy-les-Moulineaux (FR)
(72) Inventeur: KIRSZENBLAT, Romain, 92130 Issy-les-Moulineaux (FR); EDOUARD, Paul, 92130 Issy-les-Moulineaux (FR)
(74) Mandataire: Withings IP
(86) Numéro de dépôt international: PCT/EP2023/059588
(87) Numéro de publication internationale: WO 2023/208586

(56) Documents cités:
- CN-A- 114 343 596
- JP-A- 2013 183 845
- US-A1- 2012 016 210
- US-A1- 2014 276 149
- US-A1- 2019 387 985
- CAO YETONG YETONGCAO@BIT EDU CN ET AL: "Crisp-BP continuous wrist PPG-based blood pressure measurement", PROCEEDINGS OF THE 27TH ANNUAL INTERNATIONAL CONFERENCE ON MOBILE COMPUTING AND NETWORKING, ACM, NEW YORK, NY, USA, 25 October 2021 (2021-10-25), pages 378 - 391, XP058751428, ISBN: 978-1-4503-8342-4, DOI: 10.1145/3447993.3483241

## Description

La présente description concerne les dispositifs et méthodes associées pour mesurer un signal optique pulsatile, c'est-à-dire pour mesurer par voie optique une grandeur modulée ou influencée par les pulsations cardiaques d'un individu. On peut citer par exemple les signaux exploités dans des procédés de photopléthysmographie (ou « PPG », les signaux correspondants étant dénommés PPG par la suite). En particulier, la présente description concerne le positionnement d'un dispositif portable comprenant un capteur optique (par exemple un capteur PPG), et concerne notamment le serrage dudit dispositif contre la peau d'un utilisateur. Une application réside dans les dispositifs portables (dénommés *« wearable devices »* en anglais), tels que les montres ou trackers connectés avec capteur optique.

### Etat de la technique

Un capteur PPG, lorsqu'il est mal positionné, génère une mesure de mauvaise qualité de la pulsatilité de la circulation sanguine. Le positionnement au poignet est en outre une source additionnelle de difficulté, du fait qu'il impose une mesure PPG en réflexion et non pas en transmission, comme le font la plupart des dispositifs au doigt. Parmi les paramètres qui influent sur la mesure, la pression de contact entre le capteur PPG et la peau a été étudiée (voir notamment « Measurement Site and Applied Pressure Consideration in Wrist Photoplethysmography », de Geun et al., in The 23rd International Technical Conference on Circuits/System, Computers and Communications) et des solutions ont été proposées.

Par exemple, le document US2017/0209053 (Fitbit Inc.) propose une méthode utilisant la qualité du signal PPG pour signifier à l'utilisateur de serrer davantage le dispositif sur le poignet. Parmi les mauvais positionnements, on peut citer la situation dans laquelle le capteur PPG appuie trop fort sur la peau et génère une mesure de mauvaise qualité. Des méthodes existent pour informer l'utilisateur d'une pression de contact trop importante. Par exemple, le document US 10874348 (Apple Inc.) propose une méthode avec un capteur de force dédié. Par exemple, le document US2020/0146629 (Huawei) propose une méthode consistant à calibrer le capteur en prenant des mesures de PPG pour différents réglages de serrage. Par exemple, le document US2020/0146630 (Samsung Electronics Co) propose une méthode utilisant une mesure de PPG pour déterminer l'état de serrage à l'aide du ratio AC/DC à une longueur d'onde donnée ; en particulier, ce document propose d'utiliser ce ratio AC/DC à plusieurs longueurs d'onde données (dans le domaine du vert, bleu, rouge, infrarouge, etc.). Par exemple, le document CN113827185 (Huawei) propose aussi d'analyser le signal PPG pour déterminer un degré de serrage. Le US2019/0387985 utilise un signal multi-longueur d'onde pour obtenir une pression de contact entre le corps et le capteur.

Ces solutions présentent chacune des difficultés fonctionnelles ou techniques : intégration d'un équipement supplémentaire dans un dispositif portable (généralement au poignet), calibration nécessaire sur chaque utilisateur, imprécision des algorithmes, dépendance aux conditions d'utilisations (température, heure de la journée, etc.).

### Résumé de l'invention

La présente description vise à proposer des méthodes et des dispositifs ou systèmes associés ne présentant pas au moins une des difficultés précitées. Plus spécifiquement, les méthodes et dispositifs de la présente description ont recours à un ou plusieurs signaux optiques, par exemple des signaux PPG. En particulier, selon un aspect de la description, des données optiques obtenues à partir de signaux optiques à différentes longueurs d'onde sont comparées entre elles, le résultat de cette comparaison étant comparé à un seuil ; selon un autre aspect de la description, des données optiques obtenues à partir de signaux optiques émis dans le domaine du vert et du rouge ou dans le domaine du vert et de l'infrarouge sont combinées ; selon un autre aspect de la description, des données optiques obtenues à partir de signaux optiques générés par des couples source de lumière/récepteurs espacés différemment sont comparées.

L'invention est définie par les revendications.

Dans un mode de réalisation, il est proposé une méthode d'évaluation d'une pression de contact entre un capteur optique et la peau d'un utilisateur, la méthode comprenant : a) détermination de premières données optiques, à l'aide d'un premier signal optique obtenu par le capteur optique à une première longueur d'onde ; b) détermination de deuxièmes données optiques, à l'aide d'un deuxième signal optique obtenu par le capteur optique à une deuxième longueur d'onde, différente de la première longueur d'onde ; c) analyse d'au moins une comparaison des premières données optiques et des deuxièmes données optiques, l'analyse générant une information relative à la pression de contact entre le capteur optique et la peau. Les signaux optiques peuvent être des signaux optiques modulés par la pulsation cardiaque. Dans ce cas, plus spécifiquement, les données optiques comprennent une information relative à la composante pulsatile du signal optique. Par exemple, la détermination des données optiques peut comprendre un traitement impliquant une extraction de données pulsatiles du signal optique. La méthode peut en outre comprendre : en fonction au moins du résultat de l'analyse, génération d'instructions de notification à l'attention de l'utilisateur, la notification incitant l'utilisateur à modifier, notamment à diminuer, la pression de contact entre la peau et le dispositif de mesure.

La comparaison peut être effectuée au moyen du calcul d'un paramètre, le paramètre étant calculé à partir des premières données optiques et des deuxièmes données optiques. Le paramètre permet de déduire une information relative à la pression de contact entre le capteur optique et la peau. Notamment, la valeur du paramètre peut être représentative de la pression de contact entre le capteur optique et la peau. Complémentairement ou alternativement à la génération d'instructions, la méthode peut comprendre le stockage de la valeur du paramètre.

Le calcul du paramètre peut comprendre le calcul d'un ratio entre les premières données optiques et les deuxièmes données optiques, de sorte que la comparaison se fait au moyen d'un rapport. Le calcul du paramètre peut alternativement ou complémentairement comprendre le calcul d'une différence entre les premières données optiques et les deuxièmes données optiques, de sorte que la comparaison comporte une soustraction ou une soustraction avec une division (pour normaliser).

L'analyse peut comprendre la comparaison de la valeur du paramètre à un seuil (le paramètre est donc comparé à un seuil). Dans ce cas, la génération d'instructions peut être mise en œuvre en réponse à ladite comparaison (par exemple, si la valeur du paramètre est inférieure ou supérieure audit seuil). Le seuil peut notamment être fixé en fonction des caractéristiques du capteur optique et des longueurs d'onde utilisées. Le seuil peut être compris entre 0.5 et 10, par exemple entre 0.5 et 2.

La première longueur d'onde peut être inférieure à 600 nm et la deuxième longueur d'onde peut être strictement supérieure à 600 nm, voire supérieure à 650 nm.

La première longueur d'onde peut être comprise entre 400 nm et 600 nm et la deuxième longueur d'onde peut être comprise entre 600 nm et 1000 nm.

L'écart entre la première longueur d'onde et la deuxième d'onde peut être d'au moins 50 nm.

La première longueur d'onde peut être inférieure à 540 nm et la deuxième longueur d'onde peut être supérieure à 650 nm ou supérieure à 800 nm.

La première longueur d'onde peut être comprise entre 480 et 540 nm et la deuxième longueur d'onde peut être comprise entre 650 et 665 nm.

La méthode d'évaluation peut comprendre une étape de détermination de troisièmes données optiques, à l'aide d'un troisième signal optique obtenu par le capteur optique à une troisième longueur d'onde, différente de la première longueur d'onde et de la deuxième longueur d'onde, et l'analyse comprend une analyse des premières données optiques, des deuxièmes données optiques et des troisièmes données optiques. L'analyse peut notamment comprendre l'analyse d'une comparaison des premières données optiques avec les deuxièmes données optiques et l'analyse d'une comparaison des premières données optiques avec les troisièmes données optiques. En particulier, les deux comparaisons peuvent être effectuées au moyen du calcul de deux paramètres, de façon analogue à la comparaison décrite précédemment pour un paramètre unique. L'analyse peut comprendre la comparaison de la valeur de chacun des paramètres à un seuil. Un seuil identique pour les deux paramètres peut être utilisé, ou deux seuils (un pour chaque paramètre) peuvent être utilisés. La troisième longueur peut être supérieure à 800 nm, et par exemple comprise entre 800 nm et 1000 nm, ou par exemple comprise entre 920 nm et 960 nm.

La méthode peut comprendre une étape de lancement d'une mesure optique à l'aide du capteur optique et une étape d'interruption de la mesure optique en fonction au moins d'un résultat de l'analyse, l'analyse ayant généré une information relative à la pression de contact entre le capteur optique et la peau.

Dans un mode de réalisation, il est proposé une méthode d'analyse optique à l'aide d'un capteur optique configuré pour être au contact de la peau d'un utilisateur, la méthode comprenant les étapes suivantes : a) détermination de premières données optiques, à l'aide d'un premier signal optique obtenu par le capteur optique à une première longueur d'onde inférieure à 600 nm, b) détermination de deuxièmes données optiques, à l'aide d'un deuxième signal optique obtenu par le capteur optique à une deuxième longueur d'onde supérieure à 650 nm ; c) analyse d'une comparaison des premières données optiques et des deuxièmes données optiques, l'analyse comprenant un calcul d'un paramètre. En particulier, la comparaison peut être effectuée au moyen du calcul d'un paramètre à partir des premières données optiques et des deuxièmes données optiques. Le calcul du paramètre peut comprendre (voire être) le calcul d'un ratio entre les premières données optiques et les deuxièmes données optiques (ou le ratio inverse).

Dans un mode de réalisation, il est proposé une méthode d'évaluation d'une pression de contact entre un capteur optique et la peau d'un utilisateur, la méthode comprenant : a) détermination de premières données optiques, à l'aide d'un premier signal optique obtenu par le capteur optique à une première longueur d'onde ; b) détermination de deuxièmes données optiques, à l'aide d'un deuxième signal optique obtenu par le capteur optique à une deuxième longueur d'onde ; c) en fonction du résultat d'une analyse d'au moins une comparaison des premières données optiques et des deuxièmes données optiques, génération d'instructions de notification à l'attention de l'utilisateur, la notification incitant l'utilisateur à modifier, notamment à diminuer, la pression de contact entre la peau et le dispositif de mesure.

Dans un mode de réalisation, il est proposé une méthode d'évaluation d'une pression de contact entre un capteur optique et la peau d'un utilisateur, la méthode comprenant : a) détermination de premières données optiques, à l'aide d'un premier signal optique obtenu par un premier couple de source de lumière / récepteur du capteur optique, b) détermination de deuxièmes données optiques, à l'aide d'un deuxième signal optique obtenu par un deuxième couple de source de lumière / récepteur du capteur optique, la distance entre la source de lumière et le récepteur du deuxième couple étant plus importante que la distance entre la source de lumière et le récepteur du premier couple, c) analyse d'au moins une comparaison des premières données optiques et des deuxièmes données optiques, l'analyse générant une information relative à la pression de contact entre le capteur optique et la peau.

Similairement à ce qui a été décrit précédemment, des instructions de notification peuvent être générées. De la même façon, l'analyse peut se faire au moyen d'un paramètre, comme mentionné précédemment.

Dans une variante, le capteur optique comprendre une source de lumière et deux récepteurs de lumière, les récepteurs de lumière étant agencés de sorte que leur distance respective à la source de lumière soit différente. La source de lumière et un des récepteurs de lumière forment le premier couple et la source de lumière et l'autre des récepteurs de lumière forment le deuxième couple.

Dans une variante, le capteur optique comprendre deux sources de lumière et un récepteur de lumière, les sources de lumière étant agencés de sorte que leur distance respective au récepteur de lumière soit différente. Le récepteur de lumière et une des sources de lumière forment le premier couple et le récepteur de lumière et l'autre des sources de lumière forment le deuxième couple.

Dans les méthodes décrites dans la présente demande, les signaux optiques peuvent être des signaux optiques modulés par la pulsation cardiaque, par exemple des signaux de photopléthysmographie PPG. Le capteur optique peut être un capteur PPG. Les données optiques peuvent être déterminées à l'aide d'un index de perfusion.

Dans un mode de réalisation, il est proposé une utilisation d'une comparaison de données optiques obtenues à une longueur d'onde inférieure à 600 nm (par exemple correspondant au vert) d'une part, et de données optiques obtenues à une longueur d'onde supérieure à 650 nm (par exemple correspondant au rouge ou à l'infrarouge) d'autre part, pour évaluer la présence de sang dans les tissus superficiels. Les données optiques peuvent comprendre un index de perfusion. La comparaison peut être effectuée au moyen du calcul d'un ratio. La valeur de la comparaison (par exemple la valeur du ratio de ratios) en particulier peut être comparée à un seuil.

Les méthodes décrites ci-dessus peuvent être mises en œuvre par une circuiterie de contrôle comprenant une mémoire et un processeur. La circuiterie de contrôle peut faire partie d'un dispositif de mesure, comme une montre.

Dans un mode de réalisation, il est proposé un produit programme d'ordinateur comprenant des instructions qui, lorsque le programme est exécuté par une circuiterie de contrôle, conduisent celle-ci à mettre en œuvre les étapes d'au moins une méthode parmi celles décrites ci-dessus.

Dans un mode de réalisation, il est proposé un dispositif de mesure comprenant un capteur optique comprenant une source de lumière configurée pour générer de la lumière en direction de la peau d'un utilisateur et comprenant un récepteur de lumière, configuré pour détecter de la lumière provenant de la peau de l'utilisateur (en configuration d'utilisation), dans lequel le dispositif de mesure est configuré pour mettre en œuvre au moins une méthode parmi celles décrites plus haut. Le dispositif de mesure peut comprendre une circuiterie de contrôle avec un processeur, le processeur étant configuré pour mettre en œuvre au moins une méthode parmi celles décrites plus haut.

Le dispositif peut comprendre en outre des moyens de serrage, des moyens d'attache ou un bracelet permettant de maintenir le capteur optique contre la peau de l'utilisateur. La notification à l'attention de l'utilisateur décrite plus haut, lorsqu'elle est émise, comprend une indication invitant l'utilisateur à modifier le serrage du bracelet, et notamment à le desserrer. Le dispositif de mesure peut comprendre une interface utilisateur configurée pour signifier à l'utilisateur la notification.

Lorsqu'une pression trop importante est exercée sur la peau où la mesure optique est effectuée, les vaisseaux sanguins les plus superficiels subissent un reflux sanguin, le sang présent dans ces vaisseaux étant en partie ou totalement chassé. Ainsi, un signal optique obtenu à une longueur d'onde qui pénètre le plus profondément dans la peau subira une modification faible et le signal optique obtenu à une longueur d'onde qui pénètre le moins profondément dans la peau subira une modification plus significative. En matière de pulsatilité, cela se traduit notamment par une amplitude pulsatile normalisée plus faible (appelée index de perfusion ou tout simplement perfusion). De la même façon, le signal optique le plus modulé par l'hémoglobine du sang subira une modification plus significative et le signal optique le moins modulé par l'hémoglobine subira une modification plus faible. Les effets liés à la profondeur de pénétration et à l'absorption par l'hémoglobine se cumulent, en particulier pour les couples de longueurs d'onde correspondant au vert et au rouge, ou au vert et à l'infrarouge. Par exemple, les longueurs d'onde correspondant au vert sont plus fortement absorbées par l'hémoglobine et elles pénètrent peu dans la peau : il en résulte que l'index de perfusion du vert diminue fortement lorsque la pression de contact entre un capteur optique et la peau est forte. Inversement, les longueurs d'onde correspondant au rouge ou à l'infrarouge sont plus faiblement absorbées par l'hémoglobine et elles pénètrent profondément dans la peau ; et l'index de perfusion du rouge ou de l'infrarouge est par conséquent peu influencé par la pression de contact entre le capteur optique et la peau.

Le dispositif de mesure traite les signaux optiques pour générer des données optiques relatives respectivement aux deux longueurs d'onde différentes, et compare ces données. En particulier, le dispositif de mesure génère des index de perfusion et calcule un « ratio de ratios », qui peut être le rapport entre l'index de perfusion du vert sur l'index de perfusion du rouge (ou de l'infrarouge). La normalisation de l'index de perfusion du vert, qui varie fortement en fonction de la pression de contact, par l'index de perfusion du rouge, qui varie peu, permet de s'affranchir des facteurs externes de perturbation des signaux optiques : température, positionnement du capteur optique, couleur de peau, pilosité, tension, chute de circulation sanguine, luminosité ambiante, etc. Par facteurs externes, il est entendu les facteurs qui ne dépendent pas de la pression de contact entre le capteur optique et la peau.

De plus, en combinant l'effet de l'absorption plus importante pour le vert avec sa faible profondeur, la variation de l'index de perfusion du vert est d'autant plus élevée, ce qui permet au dispositif de mesure, après normalisation avec l'index de perfusion du rouge, de discriminer des niveaux de pression de contact sans calibration sur l'utilisateur. La robustesse de la méthode s'en trouve renforcée.

### Présentation des figures

Pour une meilleure compréhension des modes de réalisation décrits, des références sont faites aux figures présentées ci-dessous :
- [FIG. 1] : la [Figure 1] présente une face avant d'un exemple de dispositif de mesure conforme à un mode de réalisation de l'invention,
- [FIG. 2] : la [Figure 2] présente une face arrière de l'exemple de dispositif de mesure de la [Figure 1],
- [FIG. 3] : la [Figure 3] présente un diagramme des composants et/ou fonctions d'un dispositif de mesure conforme à un mode de réalisation de l'invention, et d'un exemple de système informatique intégrant un tel dispositif de mesure,
- [FIG. 4] : la [Figure 4] présente un exemple de courbe d'absorption en fonction de la longueur d'onde et un exemple de diagramme de profondeur de pénétration des tissus en fonction de la longueur d'onde,
- [FIG. 5] : la [Figure 5] présente un diagramme d'une méthode d'évaluation selon un aspect de la présente description,
- [FIG. 6] : la [Figure 6] présente un diagramme du détail d'une étape de la méthode d'évaluation selon un aspect de la présente description,
- [FIG. 7] : la [Figure 7] présente une courbe illustrant un signal PPG avec la composante continue et la composante pulsatile du signal PPG,
- [FIG. 8] : la [Figure 8] présente un courbe représentant un signal PPG normalisé par la composante continue et un index de perfusion, pour trois longueurs d'onde différentes, lorsqu'une pression de contact entre le capteur optique et la peau est considérée comme correcte,
- [FIG. 9] : la [Figure 9] présente un courbe représentant un signal PPG normalisé par la composante continue et un index de perfusion, pour trois longueurs d'onde différentes, lorsqu'une pression de contact entre le capteur optique et la peau est considérée comme trop importante,
- [FIG. 10] : la [Figure 10] présente un diagramme du détail d'une étape de la méthode d'évaluation selon un aspect de la description,
- [FIG. 11] : la [Figure 11] présente une courbe représentant un signal PPG normalisé par la composante continue et un paramètre vasculaire, selon un mode de réalisation de l'invention, lorsqu'une pression de contact entre le capteur optique et la peau est considérée comme correcte,
- [FIG. 12] : la [Figure 12] présente une courbe représentant un signal PPG normalisé par la composante continue et un paramètre vasculaire, selon un mode de réalisation de l'invention, lorsqu'une pression de contact entre le capteur optique et la peau est considérée comme trop importante,
- [FIG. 13] : la [Figure 13] présente un diagramme d'une méthode de mesure physiologique.

### Description détaillée

La description suivante présente différents exemples de dispositifs de mesure et de méthodes associées qui utilisent un signal optique de type PPG pour déterminer un niveau de pression de contact entre le dispositif de mesure et la peau d'un utilisateur.

Un dispositif de mesure selon la présente description est capable d'obtenir des signaux optiques et de détecter un niveau de pression de contact entre l'utilisateur et le dispositif de mesure. Le dispositif de mesure peut comprendre un boîtier, un capteur optique configuré pour détecter un signal optique (en particulier un capteur PPG configuré pour détecter un signal PPG), et un processeur logé dans le boîtier configuré pour mettre en œuvre une méthode de traitement du signal optique détecté. La méthode d'évaluation selon la présente description peut permettre d'identifier une pression trop importante entre le capteur optique et la peau, qui fait que le signal optique ne présente pas une qualité suffisante pour obtenir une mesure physiologique (par exemple le rythme cardiaque HR, le taux de saturation en oxygène du sang SpO2, la tension artérielle ou la glucométrie, etc.). Les dispositifs de mesure peuvent aussi comprendre une interface utilisateur apte à véhiculer un message à l'utilisateur. L'interface peut prendre différentes formes : écran, aiguille, vibreur, haut-parleur, etc. et le message peut être de différente nature, en fonction de l'interface : message visuel, message audio, message haptique, etc.

Le capteur optique (par exemple un capteur PPG) comprend, dans une forme simplifiée, au moins une source de lumière (par exemple une diode électroluminescente, ou LED), apte à émettre de la lumière en direction de la peau de l'utilisateur, et au moins un récepteur de lumière (par exemple un photorécepteur), apte à recevoir la lumière qui traverse, se réfléchit, se diffuse (« scattering ») ou se diffracte dans les tissus biologiques de l'utilisateur. Le signal optique dépend des tissus traversés. En particulier, un signal PPG comprend la lumière qui a été optiquement modulée par les variations volumétriques des artères, des artérioles ou des vaisseaux capillaires.

Le ou les sources de lumière peuvent émettre de la lumière à une ou plusieurs longueurs d'onde qui sont choisies en fonction du type de mesures physiologiques que le dispositif de mesure peut effectuer. Parmi les longueurs d'onde utilisés sur les dispositifs de mesure PPG, on peut citer le vert (ou le jaune), le rouge et l'infrarouge. Le ou les récepteurs de lumière peuvent recevoir de la lumière à la ou aux longueurs d'onde correspondantes. En particulier, le dispositif de mesure comprend un capteur optique apte à opérer à au moins deux longueurs d'onde.

Le capteur optique peut être positionné à n'importe quel emplacement approprié du dispositif de mesure, et notamment sur n'importe quelle face du boîtier. Lorsque le dispositif de mesure est une montre, le capteur optique est placé sur une face arrière, pour être au contact de la peau en utilisation normale.

Le signal optique mesuré par le capteur optique peut dépendre de la nature du contact entre le capteur optique et la peau, et en particulier de la pression de contact avec laquelle le capteur optique appuie sur la peau (ou inversement, la force avec laquelle l'utilisateur appuie sur le capteur optique). En effet, un dispositif de mesure avec une faible pression de contact peut donner lieu à un contact optique faible, diminuant la quantité de lumière pénétrant dans la peau et par conséquent diminuant la qualité du signal optique obtenu par le capteur optique. A l'inverse, une pression de contact trop élevée peut chasser le sang de certains vaisseaux superficiels et modifier la forme et l'amplitude du signal optique par rapport à la pression d'application à laquelle une mesure physiologique (HR, SpO2, etc.) et son algorithme ont été calibrés.

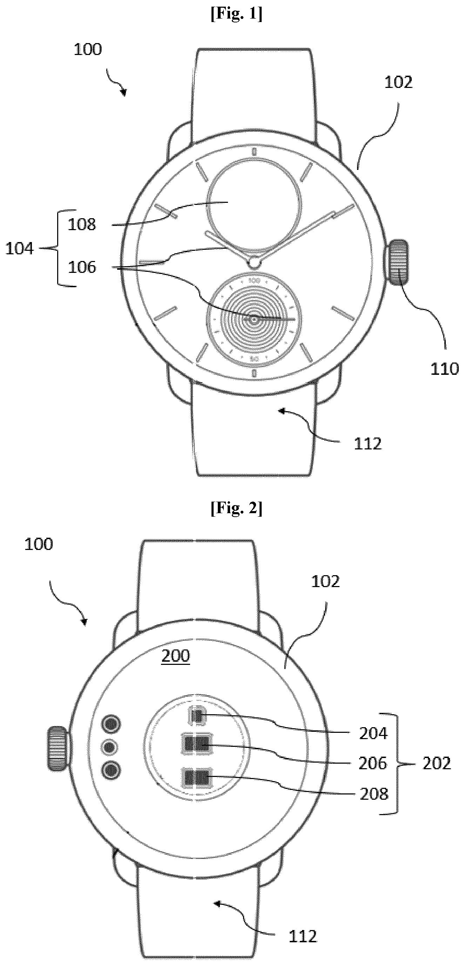

En référence à la [Figure 1] et à la [Figure 2], un exemple de dispositif de mesure 100 sous la forme d'une montre connectée est représenté. Le dispositif de mesure 100 peut comprendre un boîtier 102 et une interface utilisateur 104. L'interface utilisateur 104 a pour fonction de communiquer des informations à un utilisateur. L'interface utilisateur 104 peut comprendre des aiguilles 106 (par exemple les aiguilles qui indiquent l'heure ou une aiguille additionnelle qui peut indiquer une autre information comme le nombre de pas) et/ou un écran 108 (OLED, PMOLED, etc.). L'écran 108 peut être tactile ou non. L'interface utilisateur 104 peut comprendre, alternativement ou complémentairement, un ou plusieurs composants non visibles sur la [Figure 1], comme un vibreur ou un haut-parleur. Les aiguilles 106 et l'écran 108 permettant la transmission d'information visuelle (interface utilisateur visuelle), tandis que le haut-parleur permet la transmission d'information sonore et le vibreur permet une transmission d'information haptique (interface utilisateur haptique). D'autres moyens d'interface utilisateur peuvent être utilisés pour transmettre une information à l'utilisateur.

Le dispositif de mesure 100 peut aussi comprendre un mécanisme d'interaction 110 entre l'utilisateur et la montre. Dans l'exemple illustré, le mécanisme d'interaction 110 peut comprendre une couronne rotative et pressable, mais tout type de bouton peut être envisagé. Le mécanisme d'interaction 110 peut comprendre un écran tactile, par exemple l'écran 108, à la place ou en plus d'une couronne.

Pour attacher le dispositif de mesure 100 au poignet, des moyens d'attache ou de serrage 112 peuvent être prévus, attachés au boîtier 102, par exemple sous la forme d'un bracelet.

La [Figure 2] illustre une face arrière 200 du dispositif de mesure 100, destinée à être sur la peau de l'utilisateur. Sur cette face arrière 200, le dispositif de mesure 100 comprend un capteur optique 202, par exemple un capteur PPG. Le capteur optique 202 comprend une ou plusieurs sources de lumière 204 et un ou plusieurs récepteurs de lumière 206. Dans l'exemple illustré, le dispositif de mesure 100 comprend trois sources de lumière (dans l'exemple, des LED), représentées à 204, émettant des longueurs d'onde dans le vert, le rouge et l'infrarouge, et comprend deux récepteurs de lumières 206, 208 (dans l'exemple, des photodiodes) - l'un pour le vert et l'autre pour le rouge et l'infrarouge. Toute configuration capable de générer des signaux optiques dans deux longueurs d'onde convient. En particulier, le capteur optique peut être agencé différemment (source de lumières et récepteurs non alignés entre eux, etc.).

Lorsque le dispositif de mesure 100 est porté au poignet, la face arrière 200 est généralement au contact de la peau, ce qui signifie que le capteur optique 100 est positionné pour pouvoir émettre et recevoir de la lumière en direction de la peau, comme décrit auparavant. La pression de contact dépend notamment du serrage du moyen de serrage 112 sur le poignet de l'utilisateur.

Alternativement (non illustré), le capteur optique pourrait être disposé ailleurs sur le boîtier du dispositif de mesure et l'utilisateur pourrait simplement poser son doigt sur le capteur optique.

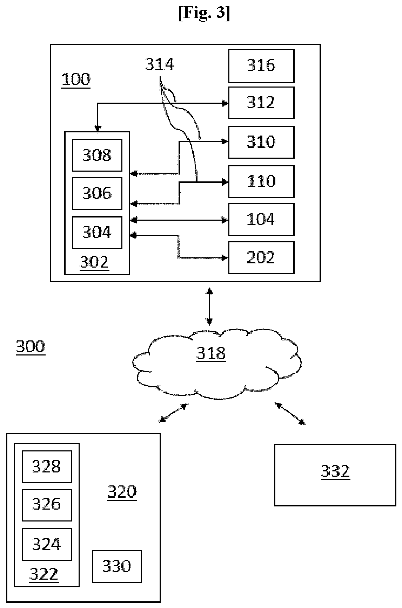

La [Figure 3] représente dans un schéma 300 les composants que peut comprendre le dispositif de mesure 100, et le système plus global dans lequel s'insère le dispositif de mesure 100. Le dispositif de mesure 100 comprend une circuiterie de contrôle 302 avec un processeur 304, une mémoire 306 et une interface I/O 308 (entrée/sortie, « input/output ») configurée pour envoyer et recevoir des données depuis/vers la circuiterie de contrôle 302. Un module de communication 310 peut être prévu pour échanger des données avec un terminal externe (par exemple un smartphone). Le module de communication 310 peut être un module sans fil, de type Wi-Fi, Bluetooth, Bluetooth Low Emission, etc. La circuiterie de contrôle 302 peut notamment échanger avec le capteur optique 202, pour obtenir des signaux optiques afin de déterminer si la pression de contact entre le capteur optique 202 et la peau d'un utilisateur est acceptable ou non, et peut activer l'interface utilisateur 104 pour notifier l'utilisateur de modifier le positionnement relatif entre la peau et le capteur optique 202.

En plus du capteur optique 202, le dispositif de mesure 100 peut comprendre différents capteurs 312 : accéléromètre, capteur GPS, compas, capteur de température, ECG (électrocardiogramme), BIA (analyse de bioimpédance « bioimpedance analysis ») etc., qui sont connectés à l'interface I/O 308. Les liaisons, par exemple électriques ou électroniques, entre les différents composants sont assurées par un ou plusieurs bus 314.

Le dispositif de mesure 100 peut comprendre une batterie 316 qui alimente les composants en énergie.

La mémoire 306 peut stocker des instructions, qui, lorsqu'exécutées par le processeur 304, mettent en œuvre la ou les méthodes de la présente description. Les méthodes sont préférablement intégralement mises en œuvre par le dispositif de mesure 100, notamment par le processeur 304 du dispositif de mesure 100 (exécution dite « en local »). Cela permet que le résultat de la mise en œuvre de ces méthodes soit disponible pour l'utilisateur sans nécessiter de connexion externe, avec notamment un terminal externe (tel qu'un smartphone).

Le dispositif de mesure 100 peut communiquer, à l'aide du module de communication 310 et à l'aide d'un réseau de communication 318, avec un terminal externe 320, de type terminal mobile intelligent (smartphone). Le terminal externe 320 comprend une circuiterie de contrôle 322 avec un processeur 324, une mémoire 326 et une interface I/O 328 configurée pour envoyer et recevoir des données depuis la circuiterie de contrôle 302. Le terminal externe 320 comprend en outre une interface utilisateur 330 pour interagir avec l'utilisateur. Le processeur 324 et la mémoire 326 peuvent mettre en œuvre une application qui permet au terminal externe 320 de communiquer avec le dispositif de mesure 100. L'interface utilisateur 330 peut notamment afficher des informations à destination de l'utilisateur.

Le dispositif de mesure 100 peut aussi communiquer avec un serveur 332, soit directement via le réseau de communication 318 soit via le terminal externe 320. Le serveur 332 peut stocker les mesures effectuées par la montre (architecture nuage de type « cloud »).

Le réseau de communication peut être hétérogène : sans-fil courte portée (Bluetooth, Wi-Fi, etc.), sans-fil longueur portée (cellulaire, etc.), câblé (Ethernet, etc.).

Dans un mode de réalisation, le dispositif de mesure100 est un dispositif portable (« wearable device » en terminologie anglaise), c'est-à-dire un dispositif fait pour être porté sur le corps humain. Dans l'exemple illustré sur la [Figure 1] et la [Figure 2], le dispositif portable 100 est une montre et la peau de l'utilisateur est la peau du poignet. Dans d'autres exemples, le dispositif de mesure 100 peut être une poignée de mesure équipant une balance de type pèse-personne, un écouteur pour oreille (« earbud » ou « earpiece »), une pince à doigt ou un boîtier destiné à d'autres usages, un patch, etc. Toutefois, les problématiques de serrage trop fort concernent généralement les dispositifs pour lesquels l'utilisateur exerce une influence directe sur la pression de contact, par exemple via le réglage des moyens de serrage.

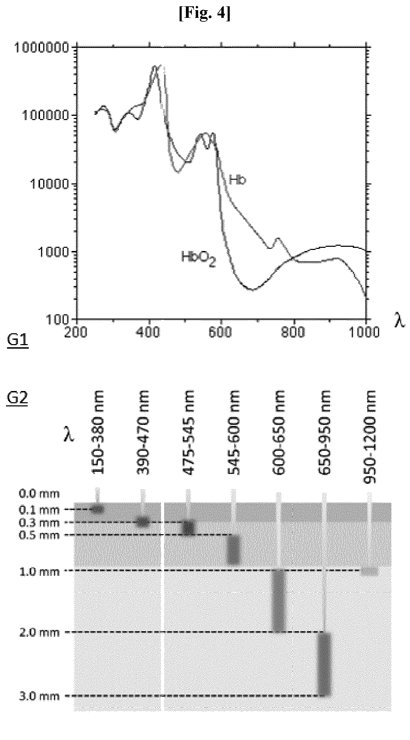

La [Figure 4] illustre deux graphiques G1 et G2 montrant les principes biophysiques impliqués dans les méthodes et dispositifs décrits dans la présente description. Le graphique G1 représente, dans une échelle logarithmique, le coefficient d'extinction molaire de l'hémoglobine et de l'oxyhémoglobine en fonction de la longueur d'onde λ. Au premier ordre, ce coefficient peut être assimilé à l'absorption : on y remarque que le vert (par exemple les longueurs d'onde inférieure à 600 nm) est nettement plus absorbé que le rouge et l'infrarouge (par exemple les longueurs d'onde supérieures à 650 nm). Le graphique G2, issu de la publication « Development of a Portable All-Wavelength PPG Sensing Device for Robust Adaptive-Depth Measurement: A Spec-trometer Approach with a Hydrostatic Measurement Example", Chang et al., in Sensors (DOI: 10.3390/s20226556), illustre la profondeur de pénétration dans la peau (en ordonnée) de différentes longueurs d'onde λ (en abscisse). Typiquement, le vert pénètre jusqu'à environ 0.3 mm, alors que le rouge et l'infrarouge pénètrent jusqu'à 3 mm.

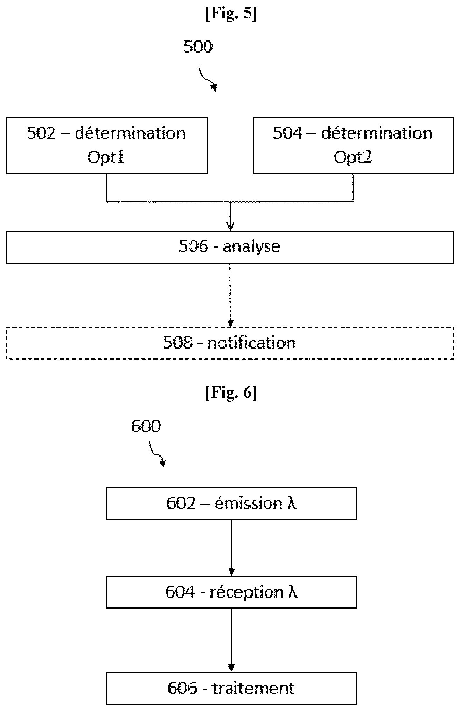

La [Figure 5] illustre les étapes d'une méthode 500 d'évaluation selon un mode de réalisation de l'invention. Cette méthode repose sur l'utilisation d'une ou plusieurs sources de lumière émettant à deux longueurs d'onde distinctes. Ces longueurs d'onde distinctes pénètrent à des profondeurs différentes dans le corps de l'utilisateur et permettent de générer des signaux optiques dont les propriétés diffèrent en fonction de la présence ou non de sang à des profondeurs différentes. La méthode 500 d'évaluation peut être mise en œuvre en relation avec une méthode de mesure physiologique : HR, SpO2, tension, glucométrie, etc.

Dans une étape 502 de détermination, la circuiterie de contrôle 302 détermine des premières données optiques à partir d'un premier signal optique obtenu par capteur optique 202 travaillant à une première longueur d'onde λ1. Dans une étape 504 de détermination, la circuiterie de contrôle 302 détermine des deuxièmes données optiques à partir d'un deuxième signal optique obtenu par le du capteur optique 202 travaillant à une deuxième longueur d'onde λ2. La deuxième longueur d'onde λ2 est différente de la première longueur d'onde λ1.

Dans un mode de réalisation, la première longueur d'onde λ1 est inférieure à 600 nm, voire inférieure à 540 nm, et la deuxième longueur d'onde λ2 est strictement supérieure à 600 nm, voire supérieure à 650 nm

Dans un mode de réalisation, la première longueur d'onde λ1 est comprise entre 500 nm et 600 nm et la deuxième longueur d'onde λ2 est comprise entre 600 nm et 1000 nm.

Dans un mode de réalisation, la première longueur d'onde λ1 est comprise entre 440 nm et 540 nm et la deuxième longueur d'onde λ2 est comprise entre 650 et 665 nm.

De plus, la différence entre la deuxième longueur d'onde λ2 et la première longueur d'onde λ1 peut être d'au moins 50 nm, afin de bénéficier davantage de l'effet lié à la différence de profondeur de pénétration entre les longueurs d'onde, comme illustré dans le graphe G2 de la [Figure 4], et/ou de bénéficier davantage de l'effet lié à l'absorption par l'hémoglobine, comme illustré dans le graphe G1 de la [Figure 4].

Dans un mode de réalisation, la première longueur d'onde λ1 est située dans le vert (ou le bleu ou le jaune) et la deuxième longueur d'onde λ2 est située dans le rouge ou l'infrarouge (infrarouge proche en particulier).

Dans un mode de réalisation, les signaux optiques sont des signaux PPG, c'est-à-dire des signaux optiques représentatifs de variations de volume. Dans un mode de réalisation, les signaux sont représentatifs de variations de vitesse. Plus généralement, les signaux optiques sont des signaux qui mesurent des caractéristiques physiques variant avec la pulsation cardiaque.

Les étapes 502 et 504 de détermination peuvent être effectuées simultanément, ou à la suite l'une de l'autre, ou bien en chevauchement. Lorsqu'elles sont effectuées à l'une suite l'une de l'autre, l'étape 502 peut être effectuée avant ou après l'étape 504.

Dans une étape 506 d'analyse, la circuiterie de contrôle 302 analyse les données optiques générées par les étapes 502, 504 de détermination. L'analyse peut comprendre une combinaison des premières données optiques et des deuxièmes données optiques afin de les comparer entre elles. Dans un mode de réalisation, l'analyse permet de générer une information relative à la pression de contact entre le capteur optique et la peau, au moyen de la comparaison entre les premières données optiques et les deuxièmes données optiques. En particulier, l'analyse peut comprendre une comparaison des premières données optiques et des deuxièmes données optiques. La comparaison peut se faire au moyen du calcul d'un paramètre dépendant notamment de la présence de sang dans les tissus superficiels (appelé paramètre vasculaire par la suite), c'est-à-dire que la valeur du paramètre permet de comparer entre elles les premières données optiques et les deuxièmes données optiques. Par exemple, la comparaison des premières données optiques et des deuxièmes données optiques peut être effectué à l'aide d'un ratio ou d'une soustraction, c'est-à-dire que le calcul du paramètre comprend respectivement le calcul d'un ratio ou d'une soustraction. L'étape 506 analyse peut permettre notamment de déterminer si une pression de contact entre la peau et le capteur de pression est trop importante, notamment pour obtenir une mesure physiologique de qualité. En pratique, les données optiques permettent de déterminer une information relative à la présence de sang dans les tissus superficiels de la peau. Le paramètre vasculaire peut donc permettre de déduire une information relative à la pression de contact entre le capteur et la peau. L'analyse 506 peut comprendre une comparaison de la valeur du paramètre vasculaire par rapport à un seuil. Le seuil, dans un mode de réalisation, peut être prédéterminé, de sorte que l'étape 506 d'analyse ne nécessite pas de calibration sur l'utilisateur.

Dans une étape 508, la circuiterie de contrôle 302 peut générer, à destination de l'interface utilisateur 104, des instructions de notification à l'attention de l'utilisateur en fonction du résultat de l'analyse de l'étape 506. La notification peut inclure une indication relative à la pression de contact entre la peau et le capteur optique 202, c'est-à-dire plus généralement entre la peau et le dispositif de mesure 100. L'indication peut avertir l'utilisateur que le capteur optique 202 appuie trop fort sur la peau. Par exemple, le dispositif de mesure 100 peut vibrer et/ou afficher un message indiquant de desserrer un bracelet.

Les étapes 502, 504 d'acquisition de données optiques et l'étape 506 d'analyse vont être décrites plus en détail.

### Détermination de données optiques

La [Figure 6] représente un diagramme 600 illustrant le détail des étapes 502, 504 de détermination, selon un mode de réalisation. Dans une étape 602 d'émission, la circuiterie de contrôle 302 instruit la source de lumière 204 du capteur optique 202 d'émettre un signal optique à une longueur d'onde donnée (λ1, λ2, etc.). Dans une étape 604 de réception, qui est concomitante à l'étape 602 du fait de la vitesse de déplacement de la lumière, la circuiterie de contrôle 302 instruit le ou les récepteurs de lumière 206, 208 du capteur optique 202 de recevoir le signal optique qui a traversé les tissus biologiques de l'utilisateur (par exemple au niveau de son poignet). A positionnement de source de lumière et de récepteur de lumière identique, le trajet effectué par la lumière dans le corps de l'utilisateur dépend de la longueur d'onde du signal optique, comme expliqué en relation avec le graphe G2 de la [Figure 4]. Enfin, dans une étape 606 de traitement, la circuiterie de contrôle 302 traite le signal optique reçu pour générer les données optiques associées au signal optique. Différents types de traitement peuvent être appliqués. Notamment, le traitement peut impliquer une extraction de données pulsatiles du signal optique reçu. Les étapes 602 d'émission, 604 de réception et 606 de traitement sont mises en œuvre pour chaque longueur d'onde utilisée dans la méthode 500 d'évaluation. Comme indiqué auparavant, le signal optique reçu peut être un signal PPG.

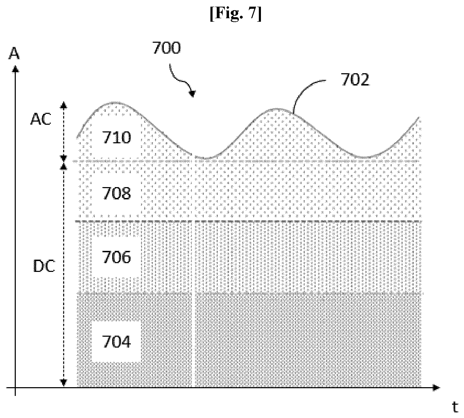

La [Figure 7] illustre une représentation graphique 700 d'un signal optique 702 reçu par le capteur optique 202, avec en abscisse le temps t et en ordonnée l'absorption A (en unité arbitraire) ; il s'agit ici d'un signal PPG. Ce signal optique 702 est décomposable en plusieurs composantes : une composante dite continue (dénommée DC), qui varie peu en fonction des pulsations du cœur, et une composante dite pulsatile (dénommée AC) qui varie plus fortement en fonction des pulsations du cœur. La composante DC correspond à l'amplitude du signal optique reçu tel que modulé par les tissus (région 704), par le sang veineux (région 706) qui est peu sujet à la pulsatilité de la circulation sanguine, et par le sang artériel non pulsatile (région 708) c'est-à-dire la quantité de sang présente en permanence dans les artères. La composante AC correspond quant à elle à la modulation par le sang artériel pulsatile de l'amplitude du signal optique reçu (région 710). Le temps entre deux pics du signal optique 702 correspond à un cycle cardiaque.

Le signal optique reçu, dont en particulier la composante continue DC de ce signal, dépend notamment de l'intensité et de la longueur d'onde du signal optique émis. Par exemple, si l'intensité lumineuse émise est accrue, alors la composante DC sera plus importante. Dans un autre exemple, en fonction de la longueur d'onde et à intensité égale de signal optique émis, l'amplitude du signal optique reçu, et en particulier l'amplitude de la composante pulsative AC, n'est pas égale, du fait des différences de valeur d'absorbance. Par conséquent, la normalisation du signal PPG ou de la composante pulsatile AC du signal PPG par la composante continue DC est généralement mise en œuvre pour s'affranchir au maximum de ces variabilités.

L'article « Signal processing and calibration improve blood measurements » de Marc Smith sur edn.com (https://www.edn.com/signal-processing-and-calibration-improve-blood-measurements/) fournit davantage d'explications sur la PPG.

### Etape 606 de traitement

L'étape 606 de traitement qui permet de générer les données optiques Opt peut être réalisée de différentes façons. Dans un mode de réalisation, la circuiterie de contrôle 302 calcule un index de perfusion (dénommé I) qui correspond au ratio de la composante pulsatile AC du signal optique reçu sur la composante continue DC du signal optique reçu. On a donc Opt=I=AC/DC. L'index de perfusion peut aussi être appelé simplement perfusion ou amplitude pulsatile normalisée (du fait de la division par la composante continue). Les composantes pulsatives et continues varient en fonction du temps, de sorte que l'on a I(t)=AC(t)/DC(t). Un index de perfusion I est calculé pour une longueur d'onde donnée, de sorte que I peut s'écrire, I_{λ}.

Par conséquent, les étapes 502 et 504 d'acquisition, au cours de laquelle la circuiterie de contrôle 302 détermine des données optiques, peuvent comprendre le traitement des signaux optiques reçus pour calculer un index de perfusion I pour chaque longueur d'onde. Les premières données optiques Opt1 sont donc l'index de perfusion I_{λ1} du signal optique reçu à la première longueur d'onde λ1 (Opt1=AC_{λ1}/DC_{λ1}) et les deuxièmes données optiques Opt2 sont l'index de perfusion I_{λ2} du signal optique reçu à la deuxième longueur d'onde λ2 (Opt1=AC_{λ2}/DC_{λ2}).

L'intérêt de l'utilisation de l'index de perfusion comme données optiques pour les méthodes et dispositifs présentement décrits est multiple. Premièrement, il s'agit d'une donnée qui est utilisée notamment pour la détermination du taux de saturation en oxygène SpO2 (voir EP3903677 par exemple). Par conséquent, son calcul à l'aide des capteurs optiques et des méthodes présentes dans les montres intelligentes (ou « *smartwatches* » en anglais) disponibles à la date de dépôt de la présente description ne pose pas de difficulté et ne génère pas d'alourdissement technique. Deuxièmement, il s'agit d'une donnée qui met en avant la pulsatilité de la circulation du sang ; en l'absence de sang, la pulsatilité est fortement réduite, ce qui est visible sur les données.

Le document EP3903677 décrit différentes techniques pour calculer les composantes AC et DC (à l'aide du filtres et/ou de soustracteurs notamment).

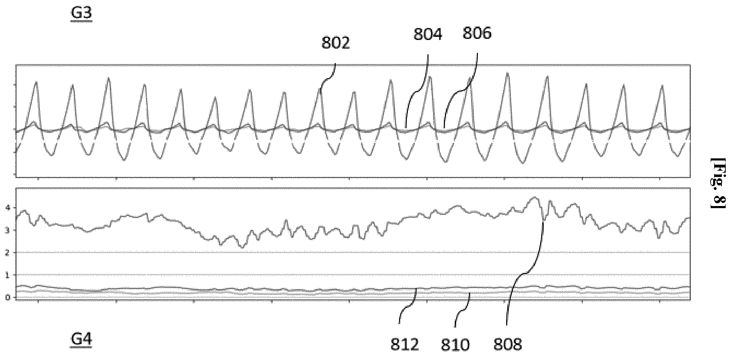

La [Figure 8] illustre une pluralité de courbes dans le cas d'un dispositif de mesure 100 avec une pression de contact considérée comme appropriée entre le capteur optique 202 et la peau : le graphe G3 illustre les signaux PPG (c'est-à-dire le signal optique reçu et filtré) normalisés par la composante continue DC de chaque signal PPG pour les longueurs d'onde dans le vert (courbe 802), le rouge (courbe 804) et l'infrarouge (courbe 806) et le graphe G4 illustre l'index de perfusion I pour les longueurs d'onde vert (courbe 808), rouge (courbe 810) et infrarouge (courbe 812). En d'autres termes, le graphe G4 illustre l'évolution de l'amplitude des courbes du graphe G3. On remarque que l'index de perfusion du signal optique vert est largement supérieur à l'index de perfusion des signaux optiques rouge et infrarouge est faible (entre 0 et 1). Les unités du graphe G3 sont arbitraires.

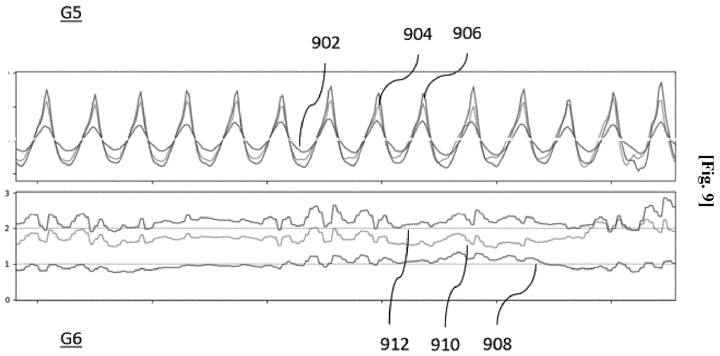

La [Figure 9] illustre une pluralité de courbes dans le cas d'un dispositif de mesure 100 avec une pression de contact trop importante entre le capteur optique 202 et la peau : le graphe G5 illustre les signaux PPG (c'est-à-dire le signal optique reçu et filtré) normalisés par la composante continue DC de chaque signal PPG pour les longueurs d'onde dans le vert (courbe 902), le rouge (courbe 904) et l'infrarouge (courbe 906) et le graphe G6 illustre l'index de perfusion I pour les longueurs d'onde dans le vert (courbe 908), le rouge (courbe 910) et l'infrarouge (courbe 912). En d'autres termes, le graphe G6 illustre l'évolution de l'amplitude des courbes du graphe G5. On y remarque que l'index de perfusion du signal optique dans le vert est inférieur à l'index de perfusion des signaux optiques dans le rouge et dans l'infrarouge. Les unités du graphe G5 sont arbitraires.

La [Figure 8] et la [Figure 9] illustrent les premières données optiques lorsque la première longueur d'onde λ1 correspond au vert et les deuxièmes données optiques lorsque la deuxième longueur d'onde λ2 correspond au rouge ou à l'infrarouge.

Dans un mode de réalisation, l'étape 606 de traitement peut comprendre une étape subséquente d'extraction des extrema des index de perfusion I et/ou de calcul d'une moyenne temporelle des index perfusion I, de sorte que les données optiques sont les extrema ou les moyennes de index de perfusion. La moyenne temporelle peut être calculée comme une moyenne sur une fenêtre glissante de quelques secondes (par exemple d'une à trois secondes). Dans un mode de réalisation, l'étape 606 de traitement peut comprendre le calcul d'une moyenne temporelle de la valeur absolue de l'index de perfusion I. Cette étape subséquente permet d'éviter de travailler avec l'intégralité des index de perfusion, et donc d'économiser les ressources (processeur, mémoire, batterie, qui doivent être optimisés). On utilisera aussi le terme d'index de perfusion I pour couvrir les données telles que traitées par ladite étape subséquente.

D'autres méthodes de traitement de signal permettant d'extraire la pulsatilité du signal optique peuvent convenir.

Alternativement, les données optiques peuvent être générées de façons différentes de celles décrites plus haut. Par exemple, l'étape 606 de traitement peut simplement comprendre l'extraction de la composante pulsatile AC. Si les signaux optiques sont suffisamment différents entre deux longueurs d'onde, la composante pulsatile AC peut suffire pour les discriminer : on peut alors utiliser Opt1=AC_{λ1} et Opt2=AC_{λ2}. De la même façon, la composante continue DC seule pour suffire, puisque la composante DC tend à baisser lorsque le sang est chassé des tissus superficiels : on peut alors utiliser Opt1=DC_{λ1} et Opt2=DC_{λ2}. D'autres grandeurs, moins usuelles, peuvent être utilisées, comme AC(t)/DC(t0) où t0 est un temps donné et AC(t) est la composante pulsatile qui varie en fonction du temps, ou bien AC(t)/avg(DC(t)), où avg(DC(t)) est la moyenne de la composante DC sur un intervalle de temps donné. On peut aussi utiliser PPG(t)/DC(t). De la même façon que décrite précédemment, une étape subséquente d'extraction d'extrema ou de calcul d'une moyenne temporelle peut être effectuée pour générer les données optiques.

### Description de l'analyse des données optiques

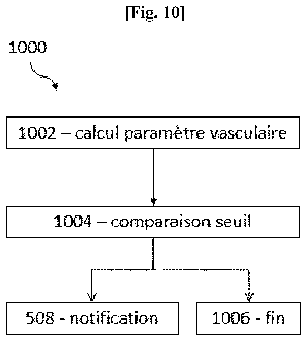

La [Figure 10] représente un diagramme 1000 illustrant le détail de l'étape 506 d'analyse selon un mode de réalisation.

Dans une étape 1002 de calcul, la circuiterie de contrôle 302 effectue la comparaison au moyen du calcul d'un paramètre vasculaire P. Le paramètre vasculaire P est une donnée obtenue à l'aide et à partir d'une combinaison des premières données optiques et des deuxièmes données optiques permettant de les comparer entre elles. Formulé autrement, si on appelle les premières données optiques Opt1 et les deuxièmes données optiques Opt2, on définit la fonction F pour calculer le paramètre P comme suit : F:(Opt1, Opt2) => F(Opt1, Opt2) = P, dont la valeur est un nombre réel. La fonction F est une fonction choisie pour comparer les premières données optiques avec les deuxièmes données optiques (et inversement). En particulier, la fonction F peut comprendre un ratio, de sorte que le paramètre vasculaire est entre les premières données optiques et les deuxièmes optiques, tel que P = F(Opt1, Opt2) = Opt1/Opt2 ou P = F(Opt1, Opt2) = Opt2/Opt1 (appelé « ratio de ratios »). Dans un mode de réalisation, le paramètre vasculaire est uniquement formé par le ratio (sans ajout d'autres données). L'intérêt d'un ratio est qu'il permet au dispositif de mesure d'opérer en relatif et donc d'avoir un paramètre vasculaire indépendant des facteurs extérieurs déjà mentionnés. Néanmoins, la fonction F peut inclure une soustraction, avec normalisation (par exemple (Opt1-Opt2)/Op2) ou sans normalisation (par exemple (Opt1-Opt2)). D'autres traitements peuvent être appliqué (valeur absolue, élévation au carré, etc.).

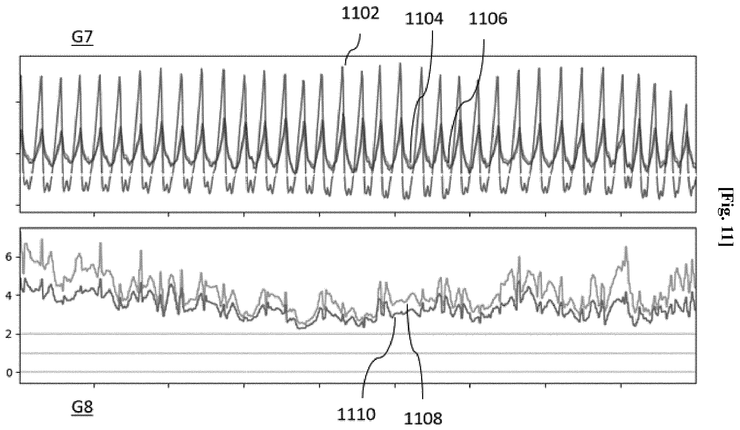

La [Figure 11] illustre une pluralité de courbes dans le cas d'un dispositif de mesure 100 avec une pression de contact considérée comme appropriée entre le capteur optique 202 et la peau : le graphe G7 (similaire au graphe G3) illustre les signaux PPG (c'est-à-dire les signaux optiques reçus et filtrés) normalisés par la composante continue DC de chaque signal PPG pour les longueurs d'onde dans le vert (courbe 1102), le rouge (courbe 1104) et l'infrarouge (courbe 1106) et le graphe G8 illustre le paramètre vasculaire sous forme du ratio de ratios (P=Opt1(vert)/Opt2(rouge ou infrarouge)) pour les longueurs d'onde vert/rouge (courbe 1108), et vert/infrarouge (courbe 1110). Les unités du graphe G7 sont arbitraires. On remarque que les paramètres vasculaires vert/rouge et vert/infrarouge sont largement supérieurs à 1.

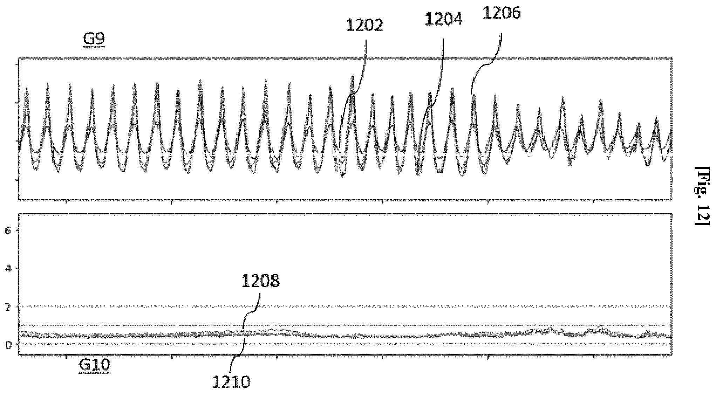

La [Figure 12] illustre une pluralité de courbes dans le cas d'un dispositif de mesure 100 avec une pression de contact trop importante entre le capteur optique 202 et la peau : le graphe G9 (similaire au graphe G5) illustre les signaux PPG (c'est-à-dire les signaux optiques reçus et filtrés) normalisés par la composante continue DC de chaque signal PPG pour les longueurs d'onde dans le vert (courbe 1202), le rouge (courbe 1204) et l'infrarouge (courbe 1206) et le graphe G10 illustre le paramètre vasculaire sous forme du ratio de ratios (P=Opt1(vert)/Opt2(rouge ou infrarouge)) pour les longueurs d'onde vert/rouge (courbe 1208), et vert/infrarouge (courbe 1210). Les unités du graphe G9 sont arbitraires. On remarque que les paramètres vasculaires vert/rouge et vert/infrarouge sont quasiment tout le temps inférieurs à 1.

Dans une étape 1004 de comparaison, la circuiterie de contrôle 302 peut effectuer une comparaison de la valeur du paramètre vasculaire par rapport à un seuil. En effet, ainsi qu'il l'a été expliqué, il est attendu que les données optiques associées à la longueur d'onde qui pénètre le moins dans les tissus soient altérées lorsqu'une pression est appliquée sur la peau à l'endroit où se situe le capteur optique 202 : cette modification se retrouve donc dans la valeur du paramètre vasculaire. La comparaison de la valeur du paramètre vasculaire par rapport à un seuil permet ainsi d'évaluer la pression appliquée sur la peau. En particulier, il a été expliqué que la composante pulsatile AC du signal optique PPG à la première longueur d'onde λ1 était fortement atténuée lorsque la pression de contact était trop importante.

Si la comparaison indique que la valeur du paramètre vasculaire P est supérieure (dans le cas où le paramètre varie dans le même sens que la pression de contact, ou respectivement inférieure s'il varie dans le sens inverse de celui de la pression de contact) à un seuil, la circuiterie de contrôle 302 peut mettre en œuvre l'étape 508 de génération d'instructions pour la notification ; si la comparaison indique que la valeur du paramètre vasculaire est inférieure (ou respectivement supérieure) au seuil, la circuiterie de contrôle 302 peut mettre fin 1006 à l'exécution la méthode. Le fait que le paramètre vasculaire variera dans le même sens ou en sens inverse de celui de la pression de contact dépend de la définition du paramètre sanguin. D'autres conditions peuvent être invoquées pour mettre en œuvre l'étape 508, par exemple des conditions liées au calcul d'un deuxième paramètre vasculaire avec une troisième longueur d'onde (voir *infra*).

La comparaison de l'étape 1004 peut se faire de différentes façons : la circuiterie de contrôle 302 peut simplement comparer la valeur du paramètre vasculaire à chaque instant ou bien à intervalles de temps réguliers, pour vérifier si au moins une valeur est au-dessus ou au-dessous du seuil ; la circuiterie de contrôle 302 peut comparer à l'aide d'une méthode d'agrégation (pour tenir compte de la proportion de la durée pendant laquelle le paramètre vasculaire est au-dessous ou au-dessus du seuil) ; la circuiterie de contrôle 302 peut comparer une moyenne du paramètre vasculaire (par exemple une moyenne sur une fenêtre glissante), etc.

Le seuil peut être prédéterminé, de sorte qu'il ne dépend pas de l'utilisateur (mais uniquement du dispositif de mesure 100 et en particulier des spécifications techniques du capteur optique 202), ce qui évite de devoir calibrer le dispositif de mesure pour chaque utilisateur.

Dans le cas du mode de réalisation où les données optiques sont un index de perfusion tel qu'illustré dans la [Figure 8] et la [Figure 9], le paramètre vasculaire P peut être un ratio entre les index de perfusion : la circuiterie de contrôle 302 peut alors comparer le ratio I_{λ1} sur I_{λ2} par rapport à un seuil K. Comme expliqué auparavant et illustré en [Figure 8] et en [Figure 9], l'index de perfusion à la première longueur d'onde λ1 (correspondant par exemple au vert) baisse significativement en cas de pression de contact trop élevée alors que l'index de perfusion à la deuxième longueur d'onde λ2 (correspondant par exemple au rouge ou à l'infrarouge) est peu impactée quelle que soit la pression de contact. La comparaison du ratio des index de perfusion avec un seuil permet d'évaluer les variations de l'index de perfusion de la première longueur d'onde λ1 en s'affranchissant des changements de conditions avec une normalisation par l'index de perfusion de la deuxième longueur d'onde λ2. En effet, l'index de perfusion peut varier en fonction de différents facteurs : température, emplacement de capteur optique sur la peau, tension artérielle, variation de circulation sanguine périphérique, couleur de peau, pilosité, etc. La normalisation permet de réduire l'influence de ces facteurs et autorise une comparaison des index avec un seuil prédéterminé. Par prédéterminé, il est signifié un seuil qui ne dépend pas de l'utilisateur (et qui peut, en cas de modification, être mis à jour via le réseau de communication 318). Cela permet une utilisation généralisée et immédiate de la méthode, sans calibration par l'utilisateur et donc sans action de sa part.

Typiquement, si la circuiterie de contrôle 302 détermine que P = I_{λ1} / I_{λ2} = (AC_{λ1}/DC_{λ1})/(AC_{λ2}/DC_{λ2}) > K, alors la pression de contact est jugée acceptable (étape 1006 de fin) ; mais si la circuiterie de contrôle 302 détermine que P = I_{λ1} / I_{λ2} ≤ K, alors la pression de contact est jugée trop importante et dans ce cas, la circuiterie de contrôle 302 peut poursuivre avec l'étape 508 de notification.

Le seuil K dépend de la configuration du capteur optique 202 et des méthodes de détermination des données optiques. Toutefois, par la nature de l'index de perfusion du vert, du rouge et/ou de l'infrarouge, le seuil K peut être choisi entre 0.5 et 5 ou entre 0.5 et 2. Dans un mode de réalisation, le seuil K est fixé à 1. Cela signifie que l'étape 506 d'analyse et en particulier l'étape 1004 de comparaison compare directement l'index de perfusion I_{λ1} de la première longueur λ1 à l'index de perfusion I_{λ2} de la deuxième longueur d'onde λ2. Quand la pression de contact est acceptable, le sang est présent en quantité normale dans les tissus superficiels et l'index de perfusion I_{λ1} de la première longueur d'onde λ1 est supérieur à l'index de perfusion I_{λ2} de la deuxième longueur d'onde λ2. Quand la pression de contact est trop importante, le sang est plus faiblement présent dans les tissus superficiels et la valeur de l'index de perfusion I_{λ1} de la première longueur d'onde λ1 devient inférieure à la valeur de l'index de perfusion I_{λ2} de la deuxième longueur d'onde λ2.

Sur l'exemple de la [Figure 11], la valeur du paramètre vasculaire (calculé pour les couples vert/IR, vert/rouge) est toujours supérieure au seuil fixé à 1. Sur l'exemple de la [Figure 12], la valeur du paramètre vasculaire (calculé pour les couples vert/IR, vert/rouge) est toujours inférieure au seuil fixé à 1.

Dans un mode de réalisation, plusieurs seuils peuvent être prévus, afin d'affiner l'évaluation de la pression de contact. Par exemple, la valeur du paramètre vasculaire peut être comparé à un premier seuil K et un deuxième seuil K', avec K'<K. En fonction de la valeur du paramètre vasculaire, notamment au moyen de ces comparaisons avec le premier seuil K et le deuxième seuil K', il est possible d'ordonner les pressions de contact et, par exemple, d'adapter les instructions à l'utilisateur. Par exemple, les instructions peuvent comprendre de desserrer légèrement le dispositif de mesure (si le paramètre est compris entre K' et K) ou de desserrer franchement le dispositif de mesure (si le paramètre est inférieur à K').

### Notification

La notification à l'attention de l'utilisateur peut être un message visuel dont le contenu indique à l'utilisateur qu'il doit diminuer la pression de contact entre le capteur optique 202 et la peau. Par exemple, la notification peut indiquer : « desserrer le bracelet », « relâcher un peu la pression », « appuyer un peu moins fort » ou bien être un dessin. Alternativement ou complémentairement, la notification peut comprendre une vibration, une lumière ou un son.

### Mode de réalisation à trois longueurs d'onde

Dans une variante, trois longueurs d'onde sont utilisées pour mettre en œuvre les méthodes décrites. Par conséquent, ce qui a été décrit pour les deux longueurs d'onde s'applique similairement à trois longueurs d'onde λ1, λ2, λ3. En particulier, la première longueur d'onde λ1 peut être inférieure à 600 nm, la deuxième longueur d'onde λ2 peut être comprise entre 600 nm (strictement au-dessus de 600 nm) et 800 nm (par exemple 655 nm), et la troisième longueur d'onde λ3 peut être comprise entre 800 et 1000 nm. Comme précédemment, il est préférable d'avoir au moins 50 nm d'écart entre la première longueur d'onde λ1 et la deuxième longueur d'onde λ2.

Dans un mode de réalisation, les longueurs d'ondes sont choisies comme suit : 480 nm < λ1 < 540 nm ; 650 nm < λ2 < 665 nm ; 920 nm < λ3 < 960 nm.

L'étape 506 d'analyse comprend alors une double comparaison entre, d'une part, les premières données optiques relatives à la première longueur d'onde λ1 et les deuxièmes données optiques relatives à la deuxième longueur d'onde λ2, et, d'autre part, les premières données les données optiques relatives à la première longueur d'onde λ1 et des troisièmes données optiques relatives à la troisième longueur d'onde λ3. La circuiterie de contrôle 302 calcule alors deux paramètres vasculaires (le premier paramètre vasculaire P12 à partir des premières données optiques et des deuxièmes données optiques, le second paramètre vasculaire P13 à partir des premières données optiques et des troisièmes données optiques).

Dans le cas de données optiques sous forme d'un ratio d'index de perfusion I, l'étape 506 peut comprendre une double comparaison : si P12 = I_{λ1} / I_{λ2} < K12 et si P13 = I_{λ1} / I_{λ3} < K13 (où K12 et K13 sont deux seuils, par exemple prédéterminés et non-dépendants de l'utilisateur) alors la pression de contact est jugée trop importante et la circuiterie de contrôle 302 peut poursuivre avec l'étape 508 de notification. Dans un mode de réalisation, on a K12=K13=K. Le choix d'imposer que, lors de l'étape 506, chaque comparaison doit être vérifiée pour évaluer la pression de contact comme trop importante permet de rendre la méthode d'évaluation plus robuste. Alternativement, la pression de contact peut être évaluée comme trop importante si au moins l'une des comparaisons est vérifiée si l'on souhaite une méthode d'évaluation plus permissive (ce qui revient à remplacer le « et » un « ou » dans la formule donnée ci-dessus).

Dans un mode de réalisation, le paramètre vasculaire est stocké dans la mémoire pour utilisation ultérieure, par exemple pour évaluer la qualité d'une mesure. Dans ce cas, le dispositif de mesure peut ne pas envoyer de notification à l'utilisateur, de sorte que la méthode 500 d'évaluation ne comprend aucune interaction avec l'utilisateur (pas d'étape 508 de notification).

### Application à une méthode de mesure

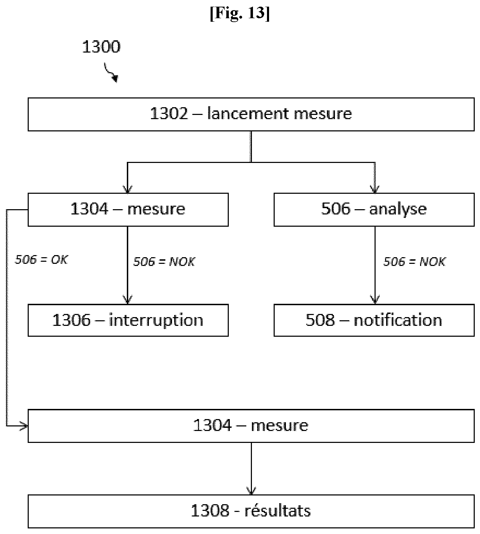

La méthode 500 d'évaluation telle que décrite précédemment peut s'intégrer dans une méthode 1300 de mesure physiologique, représentée par le diagramme de la [Figure 13]. Dans une étape 1302 de lancement de mesure, la circuiterie de contrôle 302 peut lancer une mesure optique (par exemple une mesure du taux de saturation en oxygène SpO2) et commencer à générer des données optiques. Pour une mesure de SpO2, la circuiterie de contrôle 302 calcule le ou les index de perfusion pour un ou plusieurs longueurs d'onde utilisées (vert, rouge et/ou infrarouge). Par conséquent, la méthode 500 d'évaluation telle que décrite ici peut réutiliser ces index de perfusion. Ainsi, les étapes 502, 504 d'acquisition sont effectuées, au moins partiellement, par l'étape 1302 de lancement de mesure. Pendant que, dans une étape 1304 de mesure, la circuiterie de contrôle 302 continue la mesure, la circuiterie de contrôle 302 met en œuvre l'étape 506 d'analyse pour déterminer si la pression de contact est trop importante. Quand l'étape 506 d'analyse conclut à une pression de contact trop serrée (indiqué comme « 506 = NOK » sur la [Figure 13]), la circuiterie de contrôle 302 peut mettre en œuvre l'étape 508 de génération d'instructions de notification en même temps qu'elle interrompt 1306 la mesure. Quand l'étape 506 d'analyse conclut à une pression de contact satisfaisante (indiqué comme « 506 = OK » sur la [Figure 13]), la circuiterie de contrôle 302 laisse l'étape 1304 de mesure continuer. Dans une étape 1308 de génération de résultats, la circuiterie de contrôle 302 détermine une donnée physiologique à partir de la mesure.

Alternativement, la méthode 500 d'évaluation peut être mise en œuvre indépendamment de toute mesure physiologique.

### Mode de réalisation avec différentes distances entre source de lumière et récepteur

Alternativement ou complémentairement à l'utilisateur de plusieurs longueurs d'onde différente, la méthode d'évaluation peut recourir à un capteur optique comprenant deux couples source de lumière/récepteur de lumière avec des distances différentes. Dans une variante, le capteur optique comprend une source de lumière émettant à une longueur d'onde donnée et deux récepteurs de lumière disposés agencés de sorte que leur distance respective à la source de lumière soit différente (par exemple dans un agencement aligné). Dans une variante, le capteur optique comprend deux sources de lumière émettant à une longueur d'onde (les longueurs d'ondes respectives des deux sources de lumières pouvant être identiques ou différentes) et un récepteur de lumière, agencés de sorte que leur distance respective au récepteur de lumière soit différente (par exemple dans un agencement aligné). Dans les deux variantes, le chemin optique parcouru par la lumière pour aller de la source de lumière au capteur optique le plus éloigné est plus profond que le chemin optique parcouru par la lumière pour aller de la source de lumière au capteur optique le plus proche. On obtient ainsi des premières données optiques et des deuxièmes données optiques, qui peuvent être traitées par les étapes décrites précédemment. Par exemple, sur la [Figure 21, la source de lumière 204 et le récepteur de lumière 206 forment un premier couple utilisé pour déterminer les premières données optiques ; et la source de lumière 204 et le récepteur de lumière 208 forment un deuxième couple utilisés pour déterminer les deuxièmes données optiques. Dans ce mode de réalisation, les deux récepteurs 206, 208 peuvent être configurés pour recevoir de la lumière à la même longueur d'onde.

La longueur d'onde utilisée peut correspondre à l'une des longueurs d'onde utilisées précédemment, de sorte que les valeurs données précédemment s'appliquent similairement.

Ce mode de réalisation faisant intervenir différentes distances entre sources de lumière et récepteurs peut être combiné avec le ou les modes de réalisation faisant intervenir différentes longueurs d'onde. En particulier, l'agencement du capteur optique peut être de sorte que le couple source de lumière/récepteur le plus proche est celui qui opère à la longueur d'onde la plus basse.

## Revendications

1. Méthode d'évaluation d'une pression de contact entre un capteur optique (202) et la peau d'un utilisateur, la méthode comprenant :
a) détermination (502) de premières données optiques, à l'aide d'un premier signal optique obtenu par le capteur optique (202) à une première longueur d'onde (λ1), le premier signal optique étant un signal optique modulé par la pulsation cardiaque et la détermination (502) comprenant un traitement (606) impliquant une extraction de données pulsatiles du premier signal optique,
b) détermination (504) de deuxièmes données optiques, à l'aide d'un deuxième signal optique obtenu par le capteur optique à une deuxième longueur d'onde (λ2), différente de la première longueur d'onde (λ1), le deuxième signal optique étant un signal optique modulé par la pulsation cardiaque et la détermination (502) comprenant un traitement (606) impliquant une extraction de données pulsatiles du deuxième signal optique,
c) analyse (506) d'au moins une comparaison des premières données optiques avec les deuxièmes données optiques, l'analyse générant une information relative à la pression de contact entre le capteur optique et la peau.

2. Méthode d'évaluation selon la revendication 1, comprenant :
d) en fonction au moins du résultat de l'analyse (506), génération (508) d'instructions de notification à l'attention de l'utilisateur, la notification incitant l'utilisateur à modifier, notamment à diminuer, la pression de contact entre la peau et le dispositif de mesure (100).

3. Méthode d'évaluation selon l'une des revendications 1 à 2, dans laquelle la comparaison est effectuée au moyen du calcul (1004) d'un paramètre à partir des premières données optiques et des deuxièmes données optiques, le paramètre permet de déduire une information relative à la pression de contact entre le capteur optique et la peau,
dans laquelle le calcul du paramètre comprend par exemple le calcul d'un ratio entre les premières données optiques et les deuxièmes données optiques.

4. Méthode d'évaluation selon la revendication 3, dans laquelle l'analyse comprend la comparaison (1004) de la valeur du paramètre à un seuil (K),
dans lequel le seuil (K) est notamment compris entre 0.5 et 10, par exemple entre 0.5 et 2.

5. Méthode d'évaluation selon l'une des revendications 1 à 4, dans laquelle chaque détermination (502, 504) comprend l'émission d'un signal optique à la longueur d'onde donnée et la réception du signal optique qui a traversé les tissus biologiques de l'utilisateur.

6. Méthode d'évaluation selon l'une des revendications 1 à 5, dans laquelle les premières données optiques sont déterminées à l'aide d'un premier index de perfusion et les deuxièmes données optiques sont déterminées à l'aide d'un deuxième index de perfusion.

7. Méthode d'évaluation selon l'une des revendications 1 à 6, dans laquelle la première longueur d'onde (λ1) est inférieure à 600 nm et la deuxième longueur d'onde (λ2) est strictement supérieure à 600 nm, voire supérieure à 650 nm.

8. Méthode d'évaluation selon l'une des revendications 1 à 7, dans laquelle l'écart entre la première longueur d'onde (λ1) et la deuxième d'onde (λ2) est d'au moins 50 nm.

9. Méthode d'évaluation selon l'une des revendications 1 à 8, dans laquelle la première longueur d'onde (λ1) est inférieure à 540 nm, par exemple comprise entre 480 nm et 540 nm, et la deuxième longueur d'onde (λ2) est supérieure à 650 nm, par exemple comprise entre 650 nm et 665 nm, ou supérieure à 800 nm, par exemple comprise entre 920 nm et 960 nm.

10. Méthode d'évaluation selon l'une des revendications 1 à 9, dans laquelle la première longueur d'onde correspond à du vert, du bleu ou du jaune, et la deuxième longueur d'onde correspond à du rouge ou de l'infrarouge.

11. Méthode d'évaluation selon l'une des revendications 1 à 10, comprenant :
détermination de troisièmes données optiques, à l'aide d'un troisième signal optique obtenu par le capteur optique à une troisième longueur d'onde (λ3),
différente de la première longueur d'onde (λ1) et de la deuxième longueur d'onde (λ2),
le troisième signal optique étant un signal optique modulé par la pulsation cardiaque et la détermination (502) comprenant un traitement (606) impliquant une extraction de données pulsatiles du troisième signal optique,
l'analyse (506) comprenant l'analyse d'une comparaison des premières données optiques et des deuxièmes données optiques et l'analyse d'une comparaison des premières données optiques et des troisièmes données optiques,
dans laquelle les comparaisons sont notamment effectuées au moyen d'un calcul (1004) d'un premier paramètre à partir des premières données optiques et des deuxièmes données optiques, et d'un calcul d'un deuxième paramètre à partir des premières données optiques et des troisièmes données optiques, les valeurs des premier et deuxième paramètres comprenant une information relative à la pression de contact entre le capteur optique et la peau, l'analyse (506) comprenant la comparaison de chacun des paramètres à un seuil,
dans laquelle la troisième longueur (λ3) est notamment supérieure à 800 nm, par exemple comprise entre 800 nm et 1000 nm, par exemple comprise entre 920 nm et 960 nm.

12. Méthode d'évaluation selon l'une des revendications 1 à 11, comprenant :
lancement (1302) d'une mesure optique à l'aide du capteur optique et interruption (1306) de la mesure optique en fonction du résultat de l'analyse (506).

13. Dispositif de mesure (100) comprenant un capteur optique (202) comprenant une source de lumière (204) configurée pour générer de la lumière en direction de la peau d'un utilisateur et un récepteur de lumière (206, 208), configuré pour détecter de la lumière provenant de la peau de l'utilisateur, dans lequel le dispositif de mesure comprend un processeur configuré pour mettre en œuvre la méthode selon l'une des revendications 1 à 12.

14. Dispositif selon la revendication 13, comprenant un bracelet (112) permettant de serrer le capteur optique (202) contre la peau de l'utilisateur ou comprenant un boitier sur lequel est disposé le capteur optique, de sorte que l'utilisateur pose son doigt sur le capteur optique.

15. Produit programme d'ordinateur comprenant des instructions qui, lorsque le programme est exécuté par le processeur du dispositif selon la revendication 13 ou 14, conduisent celui-ci à mettre en œuvre la méthode selon l'une des revendications 1 à 12.

## Patentansprüche

1. Verfahren zum Bewerten eines Kontaktdrucks zwischen einem optischen Sensor (202) und der Haut eines Benutzers, wobei das Verfahren umfasst :
a) Bestimmen (502) erster optischer Daten unter Verwendung eines ersten optischen Signals, das von dem optischen Sensor (202) bei einer ersten Wellenlänge (λ1) erhalten wird, wobei das erste optische Signal ein durch den Herzschlag moduliertes optisches Signal ist und das Bestimmen (502) eine Verarbeitung (606) umfasst, die eine Extraktion von pulsatilen Daten aus dem ersten optischen Signal beinhaltet,
b) Bestimmen (504) zweiter optischer Daten unter Verwendung eines zweiten optischen Signals, das von dem optischen Sensor bei einer zweiten Wellenlänge (λ2) erhalten wird, die sich von der ersten Wellenlänge (λ1) unterscheidet, wobei das zweite optische Signal ein durch den Herzschlag moduliertes optisches Signal ist und die Bestimmung (502) eine Verarbeitung (606) umfasst, die eine Extraktion von pulsatilen Daten aus dem zweiten optischen Signal beinhaltet,
c) Analysieren (506) von mindestens einem Vergleich der ersten optischen Daten mit den zweiten optischen Daten, wobei die Analyse eine Information bezüglich des Kontaktdrucks zwischen dem optischen Sensor und der Haut erzeugt.

2. Bewertungsverfahren nach Anspruch 1, umfassend :
d) zumindest in Abhängigkeit von dem Ergebnis der Analyse (506), Erzeugen (508) von Benachrichtigungsanweisungen an den Benutzer, wobei die Benachrichtigung den Benutzer dazu veranlasst, den Kontaktdruck zwischen der Haut und der Messvorrichtung (100) zu ändern, insbesondere zu verringern.

3. Bewertungsverfahren nach einem der Ansprüche 1 bis 2, wobei der Vergleich mittels Berechnung (1004) eines Parameters aus den ersten optischen Daten und den zweiten optischen Daten durchgeführt wird, wobei der Parameter die Ableitung einer Information über den Kontaktdruck zwischen dem optischen Sensor und der Haut ermöglicht,
wobei die Berechnung des Parameters beispielsweise die Berechnung eines Verhältnisses zwischen den ersten optischen Daten und den zweiten optischen Daten umfasst.

4. Bewertungsverfahren nach Anspruch 3, wobei die Analyse den Vergleich (1004) des Werts des Parameters mit einem Schwellenwert (K) umfasst,
wobei der Schwellenwert (K) insbesondere zwischen 0,5 und 10, z. B. zwischen 0,5 und 2, liegt.

5. Auswertungsverfahren nach einem der Ansprüche 1 bis 4, wobei jede Bestimmung (502, 504) das Senden eines optischen Signals bei der gegebenen Wellenlänge und das Empfangen des optischen Signals, das das biologische Gewebe des Benutzers durchdrungen hat, umfasst.

6. Beurteilungsverfahren nach einem der Ansprüche 1 bis 5, wobei die ersten optischen Daten unter Verwendung eines ersten Perfusionsindex bestimmt werden und die zweiten optischen Daten unter Verwendung eines zweiten Perfusionsindex bestimmt werden.

7. Auswertungsverfahren nach einem der Ansprüche 1 bis 6, wobei die erste Wellenlänge (λ1) kleiner als 600 nm ist und die zweite Wellenlänge (λ2) strikt größer als 600 nm oder sogar größer als 650 nm ist.

8. Auswertungsverfahren nach einem der Ansprüche 1 bis 7, wobei die Abweichung zwischen der ersten Wellenlänge (λ1) und der zweiten Wellenlänge (λ2) mindestens 50 nm beträgt.

9. Auswertungsverfahren nach einem der Ansprüche 1 bis 8, wobei die erste Wellenlänge (λ1) kleiner als 540 nm ist, z. B. zwischen 480 nm und 540 nm, und die zweite Wellenlänge (λ2) größer als 650 nm ist, z. B. zwischen 650 nm und 665 nm, oder größer als 800 nm ist, z. B. zwischen 920 nm und 960 nm.

10. Auswertungsverfahren nach einem der Ansprüche 1 bis 9, wobei die erste Wellenlänge Grün, Blau oder Gelb entspricht und die zweite Wellenlänge Rot oder Infrarot entspricht.

11. Auswertungsverfahren nach einem der Ansprüche 1 bis 10, umfassend :
Bestimmen dritter optischer Daten, unter Verwendung eines dritten optischen Signals, das von dem optischen Sensor bei einer dritten Wellenlänge (λ3) erhalten wird, die sich von der ersten Wellenlänge (λ1) und der zweiten Wellenlänge (λ2) unterscheidet,
wobei das dritte optische Signal ein durch den Herzschlag moduliertes optisches Signal ist, und wobei das Bestimmen (502) eine Verarbeitung (606) umfasst, die eine Extraktion von pulsatilen Daten aus dem dritten optischen Signal beinhaltet,
wobei das Analysieren (506) das Analysieren eines Vergleichs der ersten optischen Daten und der zweiten optischen Daten und das Analysieren eines Vergleichs der ersten optischen Daten und der dritten optischen Daten umfasst,
wobei die Vergleiche insbesondere mittels einer Berechnung (1004) eines ersten Parameters aus den ersten optischen Daten und den zweiten optischen Daten und
einer Berechnung eines zweiten Parameters aus den ersten optischen Daten und den dritten optischen Daten durchgeführt werden, wobei die Werte des ersten und des zweiten Parameters eine Information über den Kontaktdruck zwischen dem optischen Sensor und der Haut umfassen, wobei die Analyse (506) einen Vergleich jedes der Parameter mit einem Schwellenwert umfasst,
wobei die dritte Länge (λ3) insbesondere größer als 800 nm ist, z. B. zwischen 800 nm und 1000 nm, z. B. zwischen 920 nm und 960 nm.

12. Auswertungsverfahren nach einem der Ansprüche 1 bis 11, umfassend: Initiieren (1302) einer optischen Messung mithilfe des optischen Sensors und Abbrechen (1306) der optischen Messung in Abhängigkeit vom Ergebnis der Analyse (506).

13. Messvorrichtung (100) mit einem optischen Sensor (202), der eine Lichtquelle (204), die so konfiguriert ist, dass sie Licht in Richtung der Haut eines Benutzers erzeugt, und einen Lichtempfänger (206, 208) umfasst, der so konfiguriert ist, dass er Licht von der Haut des Benutzers erfasst, wobei die Messvorrichtung einen Prozessor umfasst, der so konfiguriert ist, dass er das Verfahren nach einem der Ansprüche 1 bis 12 implementiert.

14. Vorrichtung nach Anspruch 13, die ein Armband (112) umfasst, mit dem der optische Sensor (202) gegen die Haut des Benutzers gedrückt werden kann, oder die ein Gehäuse umfasst, auf dem der optische Sensor angeordnet ist, so dass der Benutzer seinen Finger auf den optischen Sensor legt.

15. Computerprogrammprodukt mit Anweisungen, die, wenn das Programm vom Prozessor des Geräts nach Anspruch 13 oder 14 ausgeführt wird, diesen dazu veranlassen, das Verfahren nach einem der Ansprüche 1 bis 12 auszuführen.

## Claims

1. An evaluation method of a contact pressure between an optical sensor (202) and a user's skin, the method comprising:
a) determination (502) of first optical data, using a first optical signal obtained by the optical sensor (202) at a first wavelength (λ1), the first optical signal being a heartbeat-modulated optical signal and the determination (502) comprising processing (606) involving extraction of pulsatile data from the first optical signal,
b) determination (504) of second optical data, using a second optical signal obtained by the optical sensor at a second wavelength (λ2), different from the first wavelength (λ1), the second optical signal being an optical signal modulated by the cardiac pulsation and the determination (502) comprising a processing (606) involving an extraction of pulsatile data from the second optical signal,
c) analysis (506) of at least one comparison of the first optical data with the second optical data, the analysis generating information relating to the contact pressure between the optical sensor and the skin.

2. The evaluation method according to claim 1, comprising :
d) depending at least on the result of the analysis (506), generating (508) notification instructions for the user, the notification prompting the user to modify, in particular to reduce, the contact pressure between the skin and the measuring device (100).

3. The evaluation method according to one of claims 1 to 2, wherein the comparison is carried out by means of the calculation (1004) of a parameter from the first optical data and the second optical data, the parameter making it possible to deduce information relating to the contact pressure between the optical sensor and the skin, wherein the calculation of the parameter comprises, for example, the calculation of a ratio between the first optical data and the second optical data.

4. The evaluation method according to claim 3, wherein the analysis comprises comparing (1004) the value of the parameter with a threshold (K),
wherein the threshold (K) is in particular between 0.5 and 10, for example between 0.5 and 2.

5. The evaluation method according to one of claims 1 to 4, wherein each determination (502, 504) comprises transmitting an optical signal at the given wavelength and receiving the optical signal which has passed through the user's biological tissue.

6. The evaluation method according to one of claims 1 to 5, wherein the first optical data is determined using a first perfusion index and the second optical data is determined using a second perfusion index.

7. The evaluation method according to one of claims 1 to 6, wherein the first wavelength (λ1) is less than 600 nm and the second wavelength (λ2) is strictly greater than 600 nm, or even greater than 650 nm.

8. The evaluation method according to one of claims 1 to 7, wherein the difference between the first wavelength (λ1) and the second wavelength (λ2) is at least 50 nm.

9. The evaluation method according to one of claims 1 to 8, wherein the first wavelength (11) is less than 540 nm, for example between 480 nm and 540 nm, and the second wavelength (λ2) is greater than 650 nm, for example between 650 nm and 665 nm, or greater than 800 nm, for example between 920 nm and 960 nm.

10. The evaluation method according to one of claims 1 to 9, wherein the first wavelength corresponds to green, blue or yellow, and the second wavelength corresponds to red or infrared.

11. The evaluation method according to one of claims 1 to 10, comprising :
determination of third optical data, using a third optical signal obtained by the optical sensor at a third wavelength (λ3), different from the first wavelength (λ1) and the second wavelength (λ2),
the third optical signal being an optical signal modulated by the heartbeat and the determination (502) comprising processing (606) involving extraction of pulsatile data from the third optical signal,
the analysis (506) comprising analysis of a comparison of the first optical data and the second optical data and analysis of a comparison of the first optical data and the third optical data,
wherein the comparisons are in particular carried out by means of a calculation (1004) of a first parameter from the first optical data and the second optical data, and a calculation of a second parameter from the first optical data and the third optical data, the values of the first and second parameters comprising information relating to the contact pressure between the optical sensor and the skin, the analysis (506) comprising the comparison of each of the parameters with a threshold, wherein the third length (λ3) is in particular greater than 800 nm, for example between 800 nm and 1000 nm, for example between 920 nm and 960 nm.

12. Evaluation method according to one of claims 1 to 11, comprising: initiation (1302) of an optical measurement using the optical sensor and interruption (1306) of the optical measurement depending on the result of the analysis (506).

13. A measuring device (100) comprising an optical sensor (202) comprising a light source (204) configured to generate light towards a user's skin and a light receiver (206, 208), configured to detect light from the user's skin, wherein the measuring device comprises a processor configured to implement the method according to one of claims 1 to 12.

14. A device according to claim 13, comprising a wristband (112) for clamping the optical sensor (202) against the user's skin or comprising a case on which the optical sensor is arranged, so that the user places his finger on the optical sensor.

15. A computer program product comprising instructions which, when the program is executed by the processor of the device according to claim 13 or 14, cause the latter to implement the method according to one of claims 1 to 12.
